# EUROPÄISCHE PATENTANMELDUNG

(11) **EP 4 252 792 A1**
(43) Veröffentlichungstag der Anmeldung: **04.10.2023**
(21) Anmeldenummer: 22165865.1
(22) Anmeldetag: 31.03.2022
(51) Int. Cl.: A61L 31/08, A61L 31/02, A61L 31/16, A61L 31/14

(54) **APATHOGENE BESCHICHTUNG EINES OBJEKTS, OBJEKT MIT DER APATHOGENEN BESCHICHTUNG UND VERFAHREN ZUM ANORDNEN EINER APATHOGENEN BESCHICHTUNG AUF EINEM OBJEKT**

(71) Anmelder: Siemens Aktiengesellschaft, 80333 München (DE)
(72) Erfinder: Doye, Christian, 13156 Berlin (DE); Jensen, Jens Dahl, 14050 Berlin (DE); Krüger, Ursus, 14089 Berlin (DE); Winkler, Gabriele, 13587 Berlin (DE)
(74) Vertreter: Siemens Patent Attorneys

(57) **Zusammenfassung**

Die Erfindung betrifft eine apathogene (antimikrobiell und/oder antiviral wirkende) Beschichtung einer Objekt-Oberfläche eines Objekts mit mindestens einer Anoden-Schicht mit mindestens einem Anoden-Material und mindestens einer Kathoden-Schicht mit mindestens einem Kathoden-Material, wobei die Schichten derart ausgestaltet sind, dass in Gegenwart von Feuchtigkeit ein galvanisches Element gebildet wird. Bestandteile der Krankheits-Erreger werden damit oxidativ zerstört. Dadurch wird die Vermehrung von Krankheits-Erregern an der Objekt-Oberfläche gehemmt. Eine effiziente Desinfektion der Objekt-Oberfläche ist möglich. Das mit der Beschichtung versehen Objekt ist insbesondere ein medizinisches Implantat, beispielsweise ein Stent.

## Beschreibung

Die Erfindung betrifft eine apathogene Beschichtung einer Objekt-Oberfläche eines Objekts, ein Objekt mit einer auf einer Objekt-Oberfläche angeordneten apathogenen Beschichtung und ein Verfahren zum Anordnen der apathogenen Beschichtung auf einer Objekt-Oberfläche des Objekts.

Apathogene (antipathogen bzw. antimikrobiell und/oder antiviral) wirkende Beschichtungen zum Hemmen des Wachstums von Krankheits-Erregern auf einer Oberfläche eines Objekts enthalten apathogen wirkende Substanzen wie Metalle oder Metall-Verbindungen (z.B. Metall-Oxide). Die apathogen wirkenden Substanzen werden Farben, Lacken oder Polymerwerkstoffen zugemischt und dann durch Streichen oder Sprühen auf die Objekt-Oberfläche des Objekts appliziert.

Gängige apathogen wirkende Substanzen sind Kupfer bzw. Kupferverbindungen wie Cuprit (Kupferoxid), Kupferthiocyanat, Kupferpyridin oder Silber (z.B. in Form von Silber-Nanopartikeln) bzw. Silber-Verbindungen wie Silberchlorid, Silbernitrat, Silberoxid, Silbernatrium-Hydrogenzirconiumphosphat und Silberzeolith A.

Die aus ihren Metall-Verbindungen freiwerdenden Metall-Ionen (Kupfer- oder Silber-Ionen), sowie die Silber-Nanopartikel reagieren mit schwefel- und phosphathaltigen Enzymen einer Zell-Membran (Zell-Wand) einer Zelle des Krankheits-Erregers und stören damit eine lebensnotwendige Transportfunktion der Zell-Membran. Die Metall-Ionen werden (wie z.B. essenzielle Calcium-Ionen) von der Zelle aufgenommen und an schwefel- und phosphathaltige Makromoleküle gebunden werden (z.B. an Aminosäuren von Proteinen). Die Metall-Ionen können sich auch an eine DNA (Desoxyribonukleinsäure) einer Zelle binden und damit eine Reproduktion der Zelle verhindern. Die beschriebenen Effekte führen zum Zelltod.

Kupfer- und Silberverbindungen sind aber nicht nur für Mikroorganismen und/oder Viren giftig, sondern können durch die gleichen Mechanismen auch für höheren Lebewesen gefährlich werden, insbesondere dadurch, dass sie sich in der Umwelt anreichern.

Aufgabe der vorliegenden Erfindung ist es, wie eine apathogene Beschichtung eines Objekts auszugestalten ist, so dass eine Belastung der Umwelt (z.B. durch die Verwendung eines Objekts mit der apathogenen Beschichtung oder bei der Herstellung des Objekts mit der Beschichtung) mit Metall-Ionen vermieden werden kann.

Zur Lösung der Aufgabe wird eine apathogene Beschichtung einer Objekt-Oberfläche eines Objekts mit mindestens einer Anoden-Schicht mit mindestens einem Anoden-Material und mindestens einer Kathoden-Schicht mit mindestens einem Kathoden-Material angegeben, wobei die Schichten derart ausgestaltet sind, dass in Gegenwart von Feuchtigkeit ein galvanisches Element gebildet wird.

Gemäß einem weiteren Aspekt der Erfindung wird ein Objekt mit einer auf einer Objekt-Oberfläche des Objekts angeordneten apathogenen Beschichtung angegeben, wobei durch die apathogene Beschichtung eine Vermehrung eines Krankheits-Erregers an der Objekt-Oberfläche zumindest gehemmt wird.

Schließlich wird zur Lösung der Aufgabe auch ein Verfahren zum Anordnen der apathogenen Beschichtung auf einer Objekt-Oberfläche eines Objekts mit folgenden Verfahrensschritten angegeben: a) Bereitstellen des Objekts mit der Objekt-Oberfläche und b) Anordnen der Beschichtung auf der Objekt-Oberfläche derart, dass in Gegenwart von Feuchtigkeit ein galvanisches Element gebildet wird.

Die Beschichtung wirkt apathogen. Die Ablagerung bzw. die Vermehrung und damit eine Anhäufung von Mikroben (z.B. Bakterien, Pilze oder Algen) und/oder von Viren auf der Objekt-Oberfläche des Objekts wird ganz oder teilweise unterbunden. Die Beschichtung wirkt damit krankheitsvermeidend ohne dass die Umwelt belastende Materialien (z.B. Metall-Ionen) freigesetzt werden.

Das galvanische Element ist ein elektrochemischer Reaktor. In Gegenwart von Feuchtigkeit entsteht ein für das galvanische Element benötigter Elektrolyt. Die Schichten bilden die Elektroden des galvanischen Elements.

Feuchtigkeit kann in verschiedenen Medien enthalten sein, z.B. in Luft (Luftfeuchtigkeit) oder in Ausdünstungen (Schweiß), Sekreten und Exkrementen von Lebewesen. Mit Hilfe der Feuchtigkeit in den verschiedenen Medien bildet sich aus der Beschichtung das (mikro-)galvanische Element. Es entstehen (mikro-) elektrische Felder. An der (Mikro-)Kathode werden dabei mit Hilfe von im Wasser gelöstem Sauerstoff reaktive Sauerstoff-Radikale wie Hyperoxide (Superoxide) und Hydroxyl-Radikale elektrochemisch gebildet. Nukleinbasen, die in den Nukleinsäuren DNA und RNA (Ribonukleinsäuren) enthalten und für die genetische Information verantwortlich sind, bestehen aus einem Grundgerüst heterocyclischer aromatischer Amine (Purine und Pyrimidine) mit Doppelbindungen. Die genannten Radikale weisen ungepaarte Elektronen in der äußeren Elektronenhülle auf und greifen die Doppelbindungen der Amine an, in dem sie mit den n-Elektronen ihre äußere Elektronenhülle auffüllen, um die Edelgaskonfiguration zu erreichen. Damit geht ein Verlust des Doppelbindungssystem im Ring einher und die Nukleinbasen können ihre Information zur Proteinbiosynthese nicht mehr weitergeben und die Replikation der Nukleinsäuren DNA und RNA wird unterbrochen. Die Mikroben (Mikro-Organismen) werden oxidativ abgetötet.

Beispielsweise verfügt der Mikroorganismus auch über eine proteinogene Aminosäure mit Schwefel. Der Schwefel der Aminosäure kann durch reaktive Sauerstoff-Spezies zu einem Sulfoxid oxidiert werden. Eine Hydroxyl-Gruppe einer SeitenKette einer proteinogenen Aminosäure kann mit Hilfe von reaktiven Sauerstoff-Spezien zu einer Aldehyd- oder einer Carboxyl-Gruppe oxidiert werden. In jedem Fall wird eine chemisch veränderte Aminosäure gebildet. Die chemisch veränderte Aminosäure kann nicht mehr am Aufbau lebensnotwendiger Proteine teilnehmen. Folglich stirbt der der Mikroorganismus.

Gemäß einer besonderen Ausgestaltung sind das Anoden-Material und/oder das Kathoden-Material porös. Die Elektroden des galvanischen Elements weisen Poren auf. Die Poren sind vorzugsweise offen. Dadurch wird eine reaktive Oberfläche der jeweiligen Elektrode erhöht. Zudem kann der durch die Feuchtigkeit gebildete Elektrolyt von der entsprechenden Elektrode aufgenommen werden.

Besonders vorteilhaft ist es, wenn das Anoden-Material ein Redoxpotential (Standard-Potential) von über +1 V und das Kathoden-Material ein Redoxpotential von unter -1 V aufweisen. Diese Redoxpotentiale eigenen sich besonders zum Initiieren elektrochemischer Reaktionen zum Abtöten von Krankheits-Erregern und damit zum effizienten Desinfizieren der Objekt-Oberfläche des Objekts.

Insbesondere weisen das Anoden-Material und/oder das Kathoden-Material ein elementares Metall auf. Gemäß einer besonderen Ausgestaltung ist das elementare Metall Titan. Titan wird insbesondere als Anoden-Material der Anoden-Schicht eingesetzt. Titan ist ein Beispiel für ein refraktäres Metall (unedles, hoch schmelzendes Metall). Andere Beispiele für refraktäre Metalle sind Zirkonium und Hafnium (4. Nebengruppe des Periodensystems der Elemente), Vanadium, Niob und Tantal (5. Nebengruppe) sowie Chrom, Molybdän und Wolfram (6. Nebengruppe).

In einer besonderen Ausgestaltung weisen das Anoden-Material und/oder das Kathoden-Material mindestens eine Metall-Verbindung auf. Vorzugsweise ist die Metall-Verbindung Mangan-Dioxid (Braunstein, MnO₂). Mangan-Dioxid wird insbesondere als Kathoden-Material der Kathoden-Schicht eingesetzt. Dabei ist es besonders vorteilhaft, poröses Mangan-Dioxid zu verwenden.

Im Hinblick auf das Verfahren zum Anordnen der Beschichtung auf der Objekt-Oberfläche werden vorzugweise ein Abscheiden des Anoden-Materials und ein Abscheiden des Kathoden-Materials auf der Objekt-Oberfläche umfasst. Dabei können die Schichten mittelbar oder unmittelbar auf der Objekt-Oberfläche aufgebracht werden. Mittelbar wären die Schichten beispielsweise auch dann aufgebracht, wenn sie übereinander an der Objekt-Oberfläche angeordnet sind. Wenn die Schichten nebeneinander angeordnet sind, könnten die Schichten auch unmittelbar auf die Objekt-Oberfläche bzw. mehrere Objekt-Oberflächen aufgebracht sein.

Mit dem Abscheiden des Anoden-Material bzw. mit dem Abscheiden des Kathoden-Materials entsteht also eine apathogene Beschichtung, mit der Folge, dass zumindest eine der Schichten mindestens eine Abscheidung aufweist.

In Abhängigkeit von der Art der Abscheidung kann jede der Schichten unterschiedliche Schichtdicken beispielsweise im µm- oder im nm-Bereich aufweisen. Gemäß einer besonderen Ausgestaltung weist zumindest eine der Schichten eine aus dem Bereich von 1 nm bis 100 µm und insbesondere eine aus dem Bereich von 10 nm bis 10 µm ausgewählte Schicht-Dicke auf.

Dabei ist es möglich, unmittelbar das entsprechende Elektroden-Material abzuscheiden. Denkbar ist aber auch, nicht das Elektroden-Material, sondern zunächst ein Ausgangs-Material (Präcursor) des Elektroden-Materials abgeschieden und anschließend das abgeschiedene Ausgangs-Material in das (eigentliche) Elektroden-Material umgewandelt wird. Gemäß einer besonderen Ausgestaltung umfassen daher das Aufbringen des Anoden-Materials und/oder das Aufbringen des Kathoden-Materials ein Aufbringen mindestens eines anodischen Ausgangs-Materials des Anoden-Materials und/oder ein Aufbringen mindestens eines kathodischen Ausgangs-Materials des Kathoden-Materials.

In einer besonderen Ausgestaltung werden zum Anordnen der Beschichtung ein physikalisches, ein chemisches und/oder ein physikalisch-chemisches Abscheideverfahren angewandt. Das physikalische Abscheideverfahren ist beispielsweise eine Kathodenstrahlzerstäubung (Sputtern), eine Elektronenstrahlverdampfung oder ein Kaltgasspritzen. Bei sehr dünnen Schichten mit Schicht-Dicken im nm- oder im subnm- (atomaren) Bereich kann ein Abscheideverfahren in Form einer chemischen Gasphasen-Abscheidung oder in Form einer Atomlagenabscheidung angewandt werden.

Beispielsweise kann Mangandioxid einerseits durch zahlreiche physikalische Verfahren und andererseits durch chemische Abscheidung auf verschiedene Materialien (Metalle, Keramik, Kunststoffe) appliziert werden. Mögliche physikalische Verfahren sind die reaktive Kathodenstrahlzerstäubung (reaktives Sputtern), Elektronenstrahlverdampfung von Mangan mit anschließender Oxidation bei 400 bis 450°C mit trockener Luft und Kaltgasspritzen.

Das Objekt ist ein beliebiger Gegenstand oder ein beliebiges Werkstück oder ein beliebiger Gebrauchs-Gegenstand, bei dem sich an einer seiner Oberflächen Krankheits-Erreger ablagern und vermehren können. Beispielsweise ist das Objekt ein alltäglich benutzter Gegenstand wie ein Treppengeländer oder ein Haltegriff eines öffentlichen Transportmittels.

Das Objekt bzw. die Objekt-Oberfläche des Objekts können dabei aus einem beliebigen Objekt-Material bestehen. Das Objekt-Material ist beispielsweise ein Metall, eine Keramik oder ein Kunststoff. Natürliche Materialien (z.B. Stein) sind auch denkbar.

Vorzugweise ist das Objekt ein medizinisches Objekt. Insbesondere ist das medizinische Objekt ein medizinisches Implantat, ein medizinisches Gerät oder ein Bedienelement des medizinischen Geräts. Das medizinische Gerät ist beispielsweise ein Operations-Besteck, ein Hilfsmittel des Operations-Bestecks oder eine diagnostische Anlage. Gerade ein medizinisches Gerät bzw. das Bedienelement des medizinischen Geräts werden in der Regel in einer keimfreien oder keimreduzierten Umgebung eingesetzt, um einer Vermehrung von Krankheits-Erregern und damit einer Infektion eines Patienten vorzubeugen.

Das medizinische Implantat ist ein in einem Körper des Patienten eingesetzter (eingepflanzter) und im Körper des Patienten temporär (über einen längeren Zeitraum) oder permanent verbleibender Gegenstand. Beispiele für solche medizinischen Implantate sind ein Herz-Schrittmacher oder eine Endo-Prothese (Gelenk-Ersatz) wie z.B. eine künstliche Hüfte. Insbesondere ist das medizinische Implantat eine Gefäß-Prothese oder ein Stent (medizinisches Implantat zum Offenhalten von Gefäßen und Hohlräumen des Körpers). Auch das medizinische Implantat muss zum Einsetzen in den Körper des Patienten steril sein.

Mit der Erfindung entfällt ein Desinfizieren des medizinischen Geräts, ein Desinfizieren des Bedienelements des medizinischen Geräts und/oder das Desinfizieren des medizinischen Implantats. Sterilität ist gewährleistet.

Die Anoden-Schicht und die Kathoden-Schicht können an der Objekt-Oberfläche des Objekts beliebig angeordnet sein. So können sie übereinander oder nebeneinander angeordnet sein. Auch können die Schichten in direktem oder indirektem Kontakt mit der Objekt-Oberfläche stehen.

Zusammenfassend sind mit der Erfindung folgende Vorteile verbunden:
- Die apathogene Beschichtung ist sehr wirksam und flexibel für verschiedenste Objekte und Anwendungen einsetzbar.
- Durch die apathogene Beschichtung werden keine giftigen Teilchen (Ionen, Atome oder Nano-Partikel) an die Umwelt abgegeben.
- Die apathogene Beschichtung ist beständig und verändert sich chemisch nicht. Die Beschichtung wirkt wie ein Katalysator, der eine chemische Reaktion erst ermöglicht oder beschleunigt, ohne dabei selbst verbraucht zu werden. Die Umwelt kann somit vor toxisch oder korrosiv wirkenden Chemikalien, die zur Dekontamination von Oberflächen oder Desinfektion von Wasser oder wässrigen Lösungen (z. B. Formaldehyd, Phenole, Hypochlorit, Cuprit, Kupferpyridin oder Silber-Nanopartikel- enthaltende Substanzen) eingesetzt werden, geschützt werden.
- Es können viele Katalyse-Zyklen durchlaufen werden (insbesondere bei der Verwendung von Titan- und MangandioxidSchichten). Antimikrobielle oder antivirale Wirkstoffe werden nicht verbraucht und müssen weder aufgefüllt oder noch ersetzt werden.
- Es entstehen keine Abfallprodukte.
- Material der Beschichtung wird nicht verbraucht. Eine Erneuerung bzw. wiederholtes Auftragen der Beschichtung ist nicht notwendig.
- Der Einsatz von Desinfektionsmitteln ist ebenso nicht notwendig.
- Bisher wurden die meisten medizinischen Implantate wie Stents aus inerten und korrosionsbeständigen Legierungen wie 316L Edelstahl, Titanlegierungen und Co-Cr- Legierungen gefertigt. Trotz des Erfolgs der Stent- Verwendung bei Behandlung von Arterienverschlüssen kann die permanente Anwesenheit in arteriellen Gefäßen zu langfristigen Komplikationen führen wie Thrombosen oder In- Stent-Restenosen. Neue Stents werden aus biologisch abbaubaren Metallen wie Eisen, Magnesium und Zink- Legierungen hergestellt. Diese temporären medizinischen Implantate mit einem sehr dünnen Titan/ Mangandioxidschichten (Schichtdicke im Nanobereich) werden gegen Mikroben- Befall geschützt. Bei Harnleiter- Stents kann durch eine dünne Ti/ MnO₂-Beschichtung die Anhaftung von Biofilmen verhindert werden.
- In der Medizin müssen Bildgebungsgeräte wie Computertomographen, Kernspintomographen, Röntgenanlagen kalibriert werden. Dazu setzt man Phantome ein; die mit Wasser gefüllt sind. Bei langer Aufbewahrung der Flüssigkeit in dem Phantom sollte die Flüssigkeitsqualität gleichbleiben. Die Besiedlung mit Keimen und Mikroorganismen kann durch eine Beschichtung der Phantom- Innenseite mit der apathogenen Beschichtung verhindert werden.
- Bedienelemente der genannten Medizingeräte sowie deren Oberflächen, mit denen das Bedienpersonal und Patienten in Berührung kommen, können zur Keimfreiheit ebenfalls mit der apathogenen Beschichtung versehen werden.
- In öffentlichen Transportmitteln wie Züge können auch die Kontaktflächen, die Fahrgäste berühren, durch die apathogenes Beschichtung vor Keimen geschützt werden. Das gilt insbesondere auch für den Sanitärbereich in Zügen.
- In der Antriebstechnik der Wasserwirtschaft werden Nieder- und Hochspannungsmotoren verwendet, die einen Luft/ Wasser Wärmeaustauscher oder einen Wassermantel zur Kühlung benötigen. Die Oberflächen, die dem Kühlwasser ausgesetzt sind, können mit der Erfindung ebenfalls vor einer Anhaftung von Mikroorganismen und der Bildung eines Biofilms geschützt werden.

Anhand von Ausführungsbeispielen und der dazugehörigen Figur wird die Erfindung näher beschrieben. Die Figur ist schematisch und stellen keine maßstabsgetreue Abbildung dar.

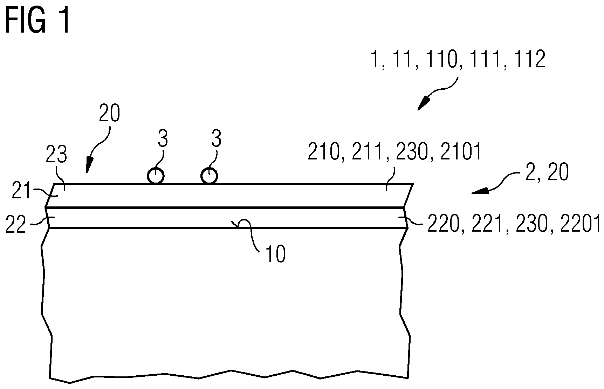

Figur 1 zeigt einen Ausschnitt eines Objekts mit apathogener Beschichtung.

Figur 2A zeigt verschiedene Oxidations-Stufen von Mangan und deren Umwandlung ineinander.

Figuren 2B bis 2D zeigen mögliche Reaktionen an der Mikro-Kathode.

Figuren 2E und 2F zeigen mögliche Reaktionen an der Mikro-Anode.

Figur 3A zeigt die Strukturformel von Methionin.

Figur 3B zeigt die Strukturformel von Serin.

Figur 3C zeigt die Oxidation von Adenin (Nukleinbase in DNA u. RNA).

Gemäß einem ersten Ausführungsbeispiel ist ein Objekt 1 in Form eines Stents (Medizinisches Objekt 11, medizinisches Implantat 110) gegeben. Gemäß einem weiteren Ausführungsbeispiel ist das Objekt 1 das Bedienelement 112 eines medizinischen Geräts 111.

Auf der Objekt-Oberfläche 10 des Objekts 1 ist eine apathogene Beschichtung 2 mit einer Anoden-Schicht (Mikro-Anode) 22 mit elementarem Titan 2201 als Anoden-Material 220 direkt auf der Objekt-Oberfläche 11 aufgebracht.

Auf der Anoden-Schicht 22 befindet sich die Kathoden-Schicht (Mikro-Kathode) 21 mit Kathoden-Material 210 in Form von porösem Mangan-Dioxid (Metall-Verbindung 2101). Die Schicht-Dicken der Anoden-Schicht 21 und der Kathoden-Schicht 22 betragen jeweils ca. 100 nm. Beide Schichten sind Gasphasen-Abscheidungen 230, wobei zum Anordnen der Mangan-Dioxid-Schicht 21 zunächst elementares Mangan als kathodisches Ausgangs-Material 211 mittels Elektronenstrahlverdampfung aufgebracht wird, das im Anschluss daran bei 400 bis 450°C oxidiert wird.

Die Schichten 21, 22 sind derart ausgestaltet, dass in Gegenwart von Feuchtigkeit 23 ein galvanisches Element (galvanisches Mikro-Element) 20 gebildet wird. Dadurch wird die Vermehrung eines Krankheits-Erregers 3 an der Objekt-Oberfläche 10 gehemmt.

Da Mangan-Dioxid nach dem elektrochemischen Standardpotential edler als Titan ist, bildet sich zwischen dem Metalldioxid Braunstein und dem Metall Titan in Gegenwart von Feuchtigkeit ein elektrisches Feld aus. Damit können an dem Braunstein und dem Titan Redox-Prozesse ablaufen und durch die dabei stattfindenden Elektronenübergänge Mikroben abgetötet werden.

Zum Anordnen der apathogenen Beschichtung 2 auf der Objekt-Oberfläche 10 wird wie folgt vorgegangen:
a) Bereitstellen des Objekts 1 mit der Objekt-Oberfläche 10 und
b) Anordnen der Beschichtung 2 an der Objekt-Oberfläche (10) derart, dass in Gegenwart von Feuchtigkeit 23 ein galvanisches Element 20 gebildet wird.

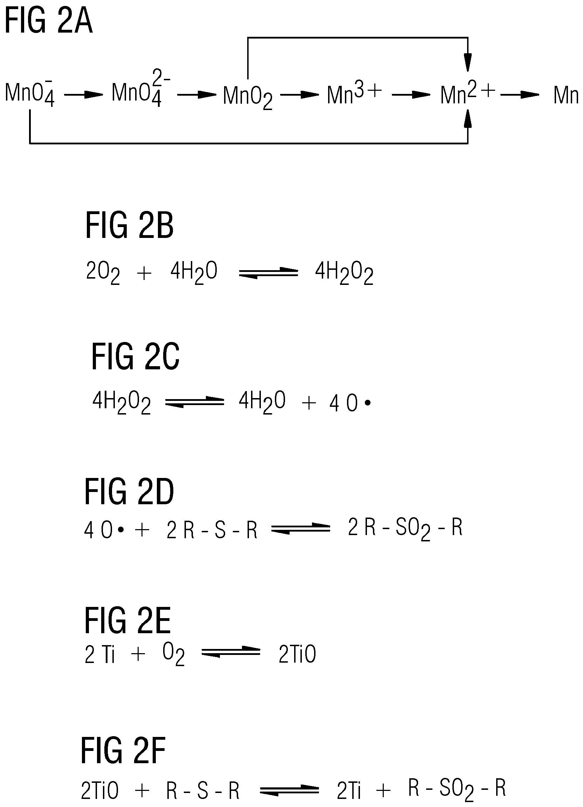

An der Mikro-Kathode lassen sich folgende Reaktionen mit den jeweiligen Standard-Potentialen identifizieren (vgl. Figur 2A) :

MnO₄⁻ → MnO₄²⁻: 0,56 V

MnO₄⁻ → Mn²⁺: 1,51 V

MnO₄²⁻ → MnO₂: 2,09 V

MnO₂ → Mn³⁺: 0,95 V

Mn³⁺ → Mn²⁺: 1,54 V

MnO₂ → Mn²⁺: 1,23 V

Mn²⁺ → Mn: -1,185 V

An der Mikro-Kathode 21 finden Reaktionen gemäß Figuren 2B, 2C und 2D statt. An der Mikro-Anode 22 treten die Reaktionen gemäß Figuren 2E und 2F auf.

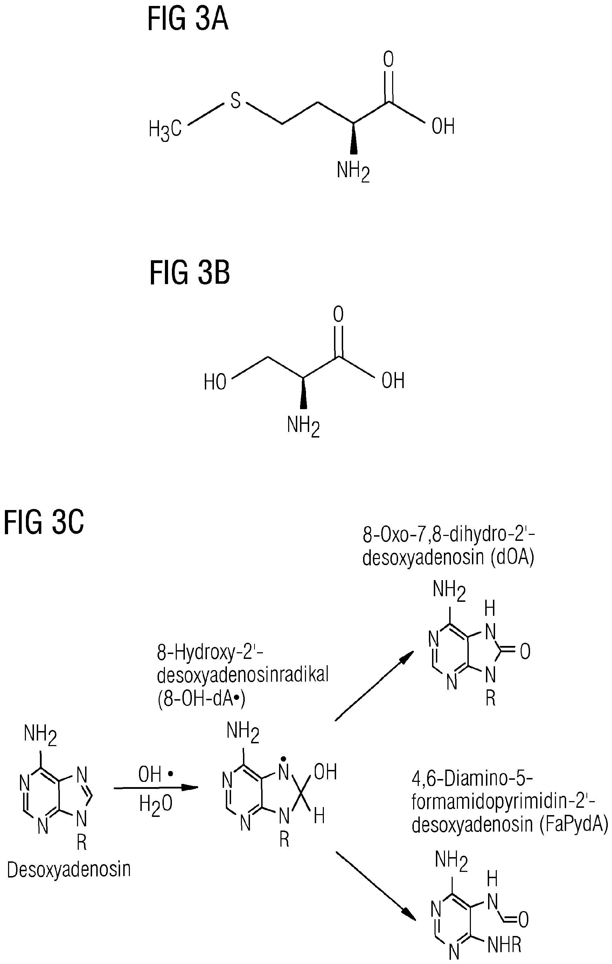

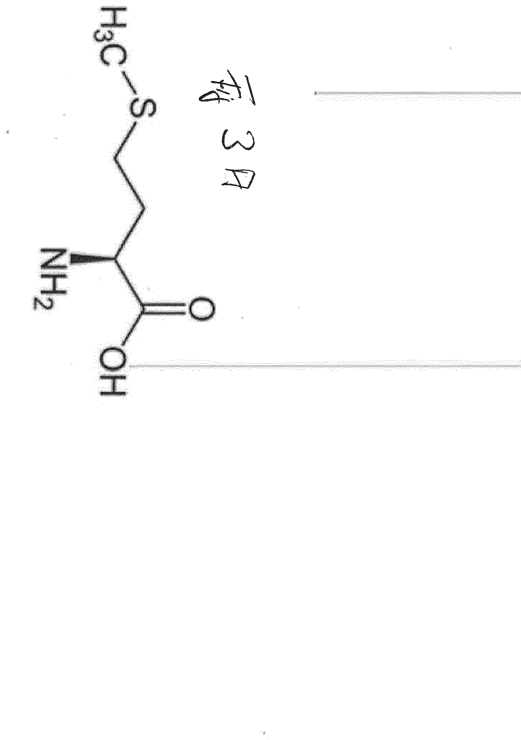

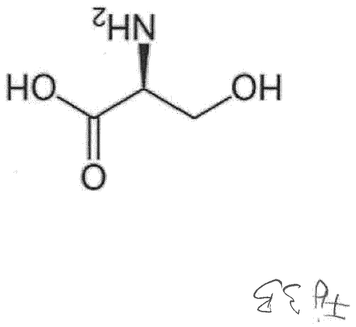

Die Figuren 3A und 3B (Methionin und Serin) sind Beispiele für proteinogene Aminosäuren, deren funktionelle Gruppen mit Hilfe der Erfindung oxidativ verändert werden können.

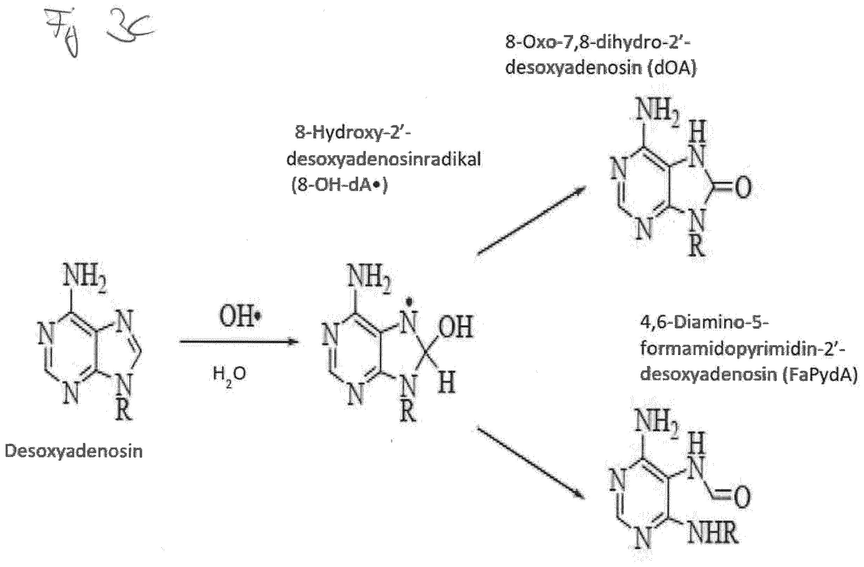

Figur 3C zeigt anhand der Oxidation von Adenin (Nukleinbase in DNA u. RNA) grundlegende Reaktionen, die mit Hilfe der Erfindung in Molekülen eines Krankheits-Erregers ausgelöst werden, sodass eine Vermehrung des Krankheits-Erregers nicht möglich ist.

## Patentansprüche

1. Apathogene Beschichtung (2) einer Objekt-Oberfläche (10) eines Objekts (1) mit
- mindestens einer Anoden-Schicht (22) mit mindestens einem Anoden-Material (220) und
- mindestens einer Kathoden-Schicht (21) mit mindestens einem Kathoden-Material (210),
wobei die Schichten (21, 22) derart ausgestaltet sind, dass in Gegenwart von Feuchtigkeit (23) ein galvanisches Element (20) gebildet wird.

2. Apathogene Beschichtung (2) nach Anspruch 1, wobei das Anoden-Material (220) und/oder das Kathoden-Material (210) porös sind.

3. Apathogene Beschichtung (2) nach Anspruch 1 oder 2, wobei das Anoden-Material (220) ein Redoxpotential von über +1 V und das Kathoden-Material (210) ein Redoxpotential von unter -1 V aufweisen.

4. Apathogene Beschichtung (2) nach einem der Ansprüche 1 bis 3, wobei das Anoden-Material (220) und/oder das Kathoden-Material (210) ein elementares Metall (2201) aufweisen.

5. Apathogene Beschichtung (2) nach Anspruch 4, wobei das elementare Metall (2201) Titan ist.

6. Apathogene Beschichtung (2) nach einem der Ansprüche 1 bis 5, wobei das Anoden-Material (220) und/oder das Kathoden-Material (210) mindestens eine Metall-Verbindung (2101) aufweisen.

7. Apathogene Beschichtung (2) nach Anspruch 6, wobei die Metall-Verbindung (2101) Mangan-Dioxid ist.

8. Apathogene Beschichtung nach einem der Ansprüche 1 bis 7, wobei zumindest eine der Schichten (21, 22) mindestens eine Abscheidung (230) aufweist.

9. Apathogene Beschichtung nach einem der Ansprüche 1 bis 8, wobei zumindest eine der Schichten (21, 22) eine aus dem Bereich von 1 nm bis 100 µm und insbesondere eine aus dem Bereich von 10 nm bis 10 µm ausgewählte Schicht-Dicke aufweist.

10. Objekt (1) mit einer auf einer Objekt-Oberfläche (10) des Objekts (1) angeordneten apathogenen Beschichtung (2) nach einem der Ansprüche 1 bis 9, wobei durch die apathogene Beschichtung (2) eine Vermehrung eines Krankheits-Erregers (3) an der Objekt-Oberfläche (10) zumindest gehemmt wird.

11. Objekt (1) nach Anspruch 10, wobei das Objekt (1) ein medizinisches Objekt (11) ist.

12. Objekt (1) nach Anspruch 11, wobei das medizinische Objekt (11) ein medizinisches Implantat (110), ein medizinisches Gerät (111) oder ein Bedienelement (112) des medizinischen Geräts (111) ist.

13. Objekt nach Anspruch 12, wobei das medizinische Implantat (110) eine Gefäß-Prothese oder ein Stent ist.

14. Verfahren zum Anordnen der apathogenen Beschichtung (2) auf einer Objekt-Oberfläche (10) eines Objekts (1) nach einem der Ansprüche 10 bis 13 mit folgenden Verfahrensschritten:
a) Bereitstellen des Objekts (1) mit der Objekt-Oberfläche (10) und
b) Anordnen der Beschichtung (2) an der Objekt-Oberfläche (10) derart, dass in Gegenwart von Feuchtigkeit (23) ein galvanisches Element (20) gebildet wird.

15. Verfahren nach Anspruch 14, wobei das Anordnen der Beschichtung (2) auf der Objekt-Oberfläche (10) ein Aufbringen des Anoden-Materials (220) und ein Aufbringen des Kathoden-Materials (210) auf der Objekt-Oberfläche (10) umfasst.

16. Verfahren nach Anspruch 15, wobei das Aufbringen des Anoden-Materials (220) ein Aufbringen mindestens eines anodischen Ausgangs-Materials (221) des Anoden-Materials (220) und/oder das Aufbringen des Kathoden-Materials (210) das Aufbringen mindestens eines kathodischen Ausgangs-Materials (211) des Kathoden-Materials (220) umfassen.

17. Verfahren nach einem der Ansprüche 14 bis 16, wobei zum Anordnen der Beschichtung (2) ein physikalisches, ein chemisches und/oder physikalisch-chemisches Abscheideverfahren angewandt werden.
